(19) Europäisches Patentamt
European Patent Office
Office européen des brevets

(11) Veröffentlichungsnummer : **0 131 130**
**B1**

(12) # EUROPÄISCHE PATENTSCHRIFT

(45) Veröffentlichungstag der Patentschrift : **23.12.87**

(51) Int. Cl.[4] : **C 07 C 35/18**, C 07 C 69/96, C 07 C175/00

(21) Anmeldenummer : **84106098.1**

(22) Anmeldetag : **29.05.84**

(54) **Verfahren zur Herstellung von Cycloalkenylalkinen.**

(30) Priorität : **09.06.83 CH 3153/83**
**13.10.83 CH 5583/83**

(43) Veröffentlichungstag der Anmeldung : **16.01.85 Patentblatt 85/03**

(45) Bekanntmachung des Hinweises auf die Patenterteilung : **23.12.87 Patentblatt 87/52**

(84) Benannte Vertragsstaaten : **AT BE CH DE FR GB IT LI NL**

(56) Entgegenhaltungen :
**DE-A- 2 558 806**
**US-A- 4 219 506**
**US-A- 4 336 401**
**LIEBIGS ANNALEN DER CHEMIE, Nr. 12, 1979, WeinheimM. BAUMANN et al. "Synthesen und einige Umsetzungen von 2,6,6-Trimethyl-2-cyclohexenon", Seiten 1945-1951**

(73) Patentinhaber : **F. HOFFMANN-LA ROCHE & CO. Aktiengesellschaft**
**CH-4002 Basel (CH)**

(72) Erfinder : **Lukàc, Teodor**
**Klusstrasse 32**
**CH-4147 Aesch (CH)**
Erfinder : **Soukup, Milan, Dr.**
**Blumenweg**
**CH-4332 Stein (CH)**
Erfinder : **Widmer, Erich, Dr.**
**Mittelweg 47**
**CH-4142 Münchenstein (CH)**

(74) Vertreter : **Zimmermann, Hans, Dr. et al**
**Grenzacherstrasse 124 Postfach 3255**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 131 130 B1

**Beschreibung**

Die vorliegende Erfindung betrifft ein neues Verfahren zur Herstellung von Cycloalkenylalkinen, welches als Zwischenstufe in Carotinoidsynthesen besonders geeignet ist. Die Erfindung betrifft ferner neue Zwischenprodukte in diesem Verfahren.

Liebigs Annalen der Chemie Nr. 12, 1945 (1979) und US-A-4 336 401 offenbaren die Herstellung strukturell ähnlicher Cycloalkenylalkine durch Wasserabspaltung. Erstere beschreibt die Herstellung von 2,6,6-Trimethyl-1-(3-methyl-3-buten-1-inyl) cyclohexen aus 2,2,6-Trimethyl-1-(3-methyl-3-buten-1-inyl) cyclohexanol, wobei die konkurrierende Rupe-Umlagerung unter milden Bedingungen unterdrückt werden konnte. US-A-4 336 401 beschreibt die Dehydratisierung von 2,6,6-Trimethyl-1-hydroxy-1-(3-acyloxy-1-butinyl)-2-cyclohexen zu 2,6,6-Trimethyl-1-(3-acyloxy-1-butinyl)-1,3-cyclohexadien bei erhöhter Temperatur.

Das erfindungsgemässe Verfahren ist dadurch gekennzeichnet, dass man eine Verbindung der allgemeinen Formel

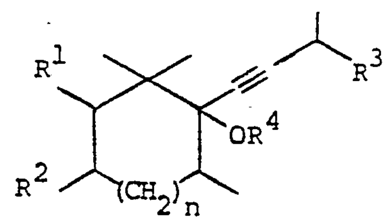

(I)

worin n für die Zahl 0 oder 1 steht; einer der Reste $R^1$ und $R^2$ Wasserstoff und der andere Wasserstoff, eine Hydroxy- oder Oxogruppe oder eine geschützte Hydroxy- oder Oxogruppe bezeichnet; $R^3$ Hydroxy, die Oxogruppe, $=CH—CH_2—OH$ oder $=CH—CHO$ oder eine von diesen Gruppen abgeleitete Gruppe mit geschützter Hydroxy- oder Oxofunktion bedeutet; $R^4$ $—CO—OR^6$, $—CO—R^6$, $—CO—NR^6R^7$, $—CO—Cl$ oder $—SO_2—R^6$ darstellt; und $R^6$ und $R^7$ gesättigte oder aromatische Kohlenwasserstoffreste oder $R^7$ auch Wasserstoff bedeuten, durch Abspaltung von $R^4OH$ in eine Verbindung der allgemeinen Formel

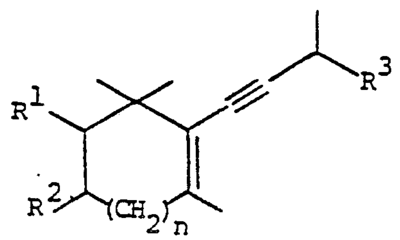

(II)

worin n, $R^1$, $R^2$ und $R^3$ die obigen Bedeutungen haben, überführt und, gewünschtenfalls, eine Verbindung der Formel II, worin $R^1$, $R^2$ oder $R^3$ eine geschützte Hydroxy- oder Oxo-gruppe aufweist, zur entsprechenden Hydroxy- oder Oxoverbindung hydrolysiert.

Der obige Ausdruck « geschützte Hydroxy- oder Oxogruppe » bzw. « geschützte Hydroxy- oder Oxofunktion » umfasst die üblicherweise für Alkohole und Ketone bzw. Aldehyde verwendeten Schutzgruppen. Bevorzugte geschützte Hydroxygruppen sind die Aether-, Silyläther- und Acetalgruppen mit bis zu 7 Kohlenstoffatomen, wie beispielsweise Methoxy, Aethoxy, Benzyloxy, Trimethylsilyloxy, (2-Methoxy-2-propyl) oxy und dergleichen. Bevorzugte geschützte Oxogruppen sind die Acetal- bzw. Ketalgruppen, mit bis zu 10 Kohlenstoffatomen, insbesondere diejenigen, worin die Oxogruppe mit einem Alkanol oder Alkandiol ketalisiert bzw. acetalisiert ist. Besonders bevorzugt bedeutet der Ausdruck geschützte Oxogruppe 2 Methoxygruppen oder die Aethylendioxygruppe.

Der Ausdruck « gesättigte oder aromatische Kohlenwasserstoffreste » umfasst Alkyl-, Aryl-, Arylalkyl- und Alkylarylreste mit vorzugsweise bis zu 10 Kohlenstoff-atomen. Beispiele für derartige Gruppen sind Methyl, Aethyl, Phenyl, Tolyl und dergleichen.

Es wurde nun gefunden, dass die Verbindungen der Formel I unter vergleichsweise milden Bedingungen und in guter Ausbeute in reine Verbindungen der Formel II übergeführt werden können.

Die erfindungsgemässe Herstellung der Verbindungen der Formel II kann in an sich bekannter Weise dadurch erfolgen, dass man in einer Verbindung der Formel I durch Erhitzen und/oder in Gegenwart eines Katalysators $R^4OH$ abspaltet. Geeignete Katalysatoren sind beispielsweise a) organische Stickstoffbasen, insbesondere primäre und sekundäre Stickstoffbasen (z. B. Imidazol, 1,2,4-Triazol, Anilin), b) Salze von organischen Stickstoffbasen mit starken Säuren (vorzugsweise Säuren mit $pK_a < 1$), insbesondere Chloride, Bromide und Tosylate (z. B. Pyridinium-p-tosylat), c) Phosphoniumsalze (z. B. Triphenylphosphoniumchlorid oder- bromid), d) Säuren, vorzugsweise starke Säuren mit $pK_a < 1$ (z. B. Toluolsulfonsäure, Amberlyste A 15® (Fluka AG), Salzsäure, Schwefelsäure), e) Trialkylchlorsilane (z. B. Trimethylchlorsilan), f) Lithiumsalze, beispielsweise Lithiumsalzevon starken ($pK_a < 1$) Säuren (z. B. Lithiumchlorid, Lithiumperchlorat, Lithiumtetrafluoroborat) und g) Palladium-(O)-Katalysatoren (z. B. durch Zusatz von Palladiumacetat).

Die Reaktionstemperatur ist insbesondere abhängig von der Bedeutung des Restes $R^4$ und davon ob die Reaktion mit oder ohne Katalysator durchgeführt wird. Im allgemeinen liegt die erforderliche Reaktionstemperatur unter 200 °C ; in einigen Fällen erfolgt die Abspaltung von $R^4OH$ jedoch schon bei Raumtemperatur. Der Druck ist nicht kritisch ; vorzugsweise wird daher bei Atmosphärendruck gearbeitet.

Die erfindungsgemässe Umsetzung einer Verbindung der Formel I erfolgt zweckmässig in einem inerten organischen Lösungsmittel, beispielsweise einem Aether, Alkohol, Nitril, Amid oder einem gesättigten, aromatischen oder chlorierten Kohlenwasserstoff. Beispiele bevorzugter Lösungsmittel sind Diäthyläther, Tetrahydrofuran, Aethylenglykoldimethyläther, Diäthylenglykoldimethyläther, Triäthylenglykoldimethyläther, Methanol, Acetonitril, Dimethylformamid, Hexan, Toluol, Benzol, Xylol, Methylenchlorid, Dichloräthan und dergleichen.

Das erfindungsgemässe Verfahren kann mit oder ohne Katalysator durchgeführt werden. Bei Verwendung eines Katalysators kann dieser in Katalytischen Mengen oder auch in höheren Mengen eingesetzt werden. Bevorzugte Katalysatoren sind die organischen Stickstoffbasen, die Salze solcher Basen und die Lithiumsalze.

Die Hydrolyse einer erhaltenen Verbindung der Formel II, worin $R^1$, $R^2$ oder $R^3$ eine geschützte Hydroxy- oder Oxo- gruppe aufweist, kann nach den an sich bekannten Methoden der Hydrolyse solcher Schutzgruppen erfolgen.

Eine weitere bevorzugte Variante des erfindungsgemässen Verfahrens besteht darin, dass man eine Verbindung der Formel I, worin $R^3$ die Gruppe $=CH-CH_2-OH$ bedeutet, in Gegenwart von äquimolaren oder höheren Mengen Phosphoniumsalz, vorzugsweise Triphenylphosphoniumchlorid oder -bromid, umsetzt. Hierbei wird zuerst die entsprechende Verbindung der Formel II gebildet, welche anschliessend direkt zum entsprechenden Phosphoniumsalz weiter umgesetzt wird (d. h. die Hydroxygruppe in $R^3$ wird in eine Phosphoniumsalz-Gruppe, z. B. $-P(C_6H_5)_3{}^{\oplus}Cl^{\ominus}$ oder $-P(C_6H_5)_3{}^{\oplus}Br^{\ominus}$ übergeführt).

Vorzugsweise werden nach dem erfindungsgemässen Verfahren Verbindungen der Formel I umgesetzt, worin n die Zahl 0 und $R^2$ Wasserstoff bedeuten oder worin n die Zahl 1 bedeutet. Besonders bevorzugt steht in Formel I n für die Zahl 1 und $R^1$ für Wasserstoff. Grundsätzlich sind ferner diejenigen Verbindungen der Formel I bevorzugt, worin einer der Reste $R^1$ und $R^2$ Wasserstoff und der andere Wasserstoff oder Hydroxy, insbesondere Hydroxy bezeichnet, wie beispielsweise die Verbindungen der Formel I, worin n die Zahl 1, $R^1$ Wasserstoff und $R^2$ Hydroxy bedeutet.

Besonders bevorzugte Reste $R^3$ sind Hydroxy und die Gruppe $=CH-CH_2-OH$. Ferner weisen in $R^3$ gegebenenfalls vorhandene Doppelbindungen generell vorzugsweise trans-Konfiguration auf.

Besonders bevorzugte Reste $R^4$ in obiger Formel I sind die Gruppen $-CO-OR^6$, insbesondere diejenigen, worin $R^6$ $C_1$-$C_6$-Alkyl bezeichnet. Ganz besonders bevorzugt sind $-CO-OCH_3$ und $-CO-OC_2H_5$. Weitere bevorzugte Reste $R^4$ in obiger Formel I sind die Gruppen $-CO-R^6$, insbesondere diejenigen, worin $R^6$ einen aromatischen Kohlenwasserstoffrest bezeichnet, z. B. Benzoyl.

Grundsätzlich sind ferner diejenigen Verbindungen der Formel I bevorzugt, worin das Wasserstoffatom in 5-Stellung des Cyclopentylringes bzw. 6-Stellung des Cyclohexylringes und die Gruppe $R^4O$- in cis-Stellung zueinander stehen (syn-Elimination).

Eine weitere bevorzugte Variante des erfindungsgemässen Verfahrens ist dadurch gekennzeichnet, dass man eine Verbindung der allgemeinen Formel

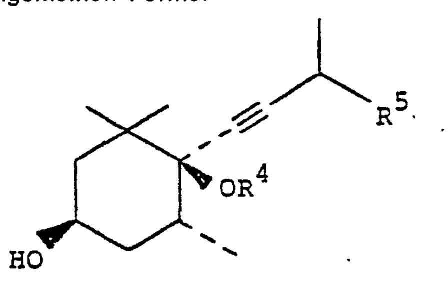

(Ia)

worin $R^5$ Hydroxy oder die Gruppe $=CH-CH_2-OH$ bezeichnet und $R^4$ die obige Bedeutung hat, oder deren optischen Antipoden durch Abspaltung von $R^4OH$ in Gegenwart einer basischen, sterisch gehinderten Lithium-verbindung in eine Verbindung der allgemeinen Formel

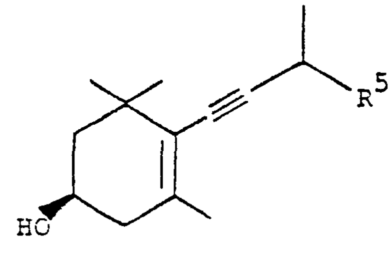

(IIa)

worin $R^5$ die obige Bedeutung hat, bzw. deren optischen Antipoden überführt.

Der Ausdruck « basische, sterisch gehinderte Lithium-verbindungen » umfasst lithium-organische Verbindungen, welche an der basischen Gruppe sterische Hinderung aufweisen (um unkleophilen Angriff an der Gruppe $—OR^4$ zu vermeiden) und von organischen Verbindungen mit $pK_a$-Werten von mindestens etwa 9, vorzugsweise mindestens etwa 11, abgeleitet sind. Die sterische Hinderung kann beispielsweise durch Verzweigung oder Substitution am Kohlenstoffatom in 1-Stellung oder durch Substitution in ortho-Stellung eines Benzolringes erfolgen. Bevorzugte derartige Verbindungen sind Alkyllithium, Lithiumalkanolate, Lithiumphenolate, Lithiumdialkylamide und dergleichen, welche die obigen Bedingungen erfüllen, beispielsweise Lithium-tert.-butylat, Lithium-1,1-dimethylpentanolat, Lithium-2,6,-dimethylphenolat, Lithium-2,6-di- tert.-butylphenolat, Lithium-2,6-dichlorphenolat, tert.-Butyllithium, 2,6-Di-tert.-butylphenyllithium and Lithiumdiisopropylamid.

Vorzugsweise werden bei dieser Variante mindestens etwa 2 Mol Lithiumverbindung pro Mol an Verbindung der Formel Ia (oder deren Antipoden) eingesetzt. Besonders bevorzugt werden etwa 2-3 Moläquivalente Lithiumverbindung verwendet; höhere Mengen wirken sich jedoch nicht nachteilig auf die Reaktion aus.

Bezüglich Temperatur, Druck und Lösungsmittel gilt das weiter oben, im Zusammenhang mit der Umsetzung der Verbindungen der Formel I Gesagte sinngemäss. Vorzugsweise wird jedoch eine Temperatur von etwa 40 °C bis etwa 70 °C angewendet. Besonders bevorzugte Lösungsmittel sind die gesättigten und aromatischen Kohlenwasserstoffe.

Diese Verfahrensvariante ergibt hohe Ausbeuten. Zudem bleibt die Konfiguration einer gegebenenfalls in $R^5$ vorhandenen Doppelbindung erhalten. Da ferner im allgemeinen nur die Verbindungen der Formel I und deren optische Antipoden (nicht aber die übrigen Stereoisomere) under den erwähnten milden Temperaturbedingungen gut reagieren, eignet sich diese Variante insbesondere zur Herstellung isomerenreiner Verbindungen.

$R^5$ in obiger Formel Ia bedeutet vorzugsweise die Gruppe $=CH—CH_2—OH$. Vorzugsweise liegt die Doppelbindung in dieser Gruppe in trans-Konfiguration vor. Bevorzugte Reste $R^4$ sind $—CO—R^6$ und insbesondere $—CO—OR^6$. Bevorzugte Beispiele derartiger Reste sind $—CO—CCH_3$, $—CO—OC_2H_5$ und $—CO—CH_3$.

Die Verbindungen der Formel I sind neu und bilden ebenfalls Gegenstand der vorliegenden Erfindung. Sie können in an sich bekannter Weise hergestellt werden, beispielsweise aus dem entsprechenden Lithiumalkoholat (Verbindung der Formel I, worin $R^4$ Lithium bedeutet) durch Umsetzung mit einem entsprechenden Chlorid, d. h. mit Verbindungen der Formeln $Cl—CO—OR^6$, $Cl—CO—R^6$, $Cl—CO—NR^6R^7$, $Cl—CO—Cl$ oder $Cl—SO_2—R^6$, worin $R^6$ und $R^7$ die obigen Bedeutungen haben. Die hierbei benötigten Lithiumalkoholate können in an sich bekannter Weise durch Umsetzung des entprechenden Cyclopentanons bzw. Cyclohexanons mit dem entsprechenden Alkinyllithium erhalten werden. Hierbei liegen weitere gegebenenfalls vorhandene Hydroxy- und Oxogruppen zweckmässig in geschützter Form vor. Derartige Schutzgruppen können dann gewünschtenfalls vor oder nach der Einführung des Restes $R^4$ oder auch erst nach der erfindungsgemässen Abspaltung von $R^4OH$ wieder abgespalten werden.

Die Umsetzung der Verbindungen der Formel II zu Carotinoiden kann in bekannter oder an sich bekannter Weise erfolgen, beispielsweise durch Ueberführung in geeignete Aldehyde oder Phosphoniumsalze und anschliessende Wittig-Reaktion.

Die Erfindung betrifft ferner alle neuen Verbindungen, Mischungen, Verfahren und Verwendungen wie hierin beschrieben.

Die Erfindung wird durch die folgenden Beispiele weiter veranschaulicht.

## Beispiel 1

10,14 g Aethyl-(1S, 4R, 6R)-4-hydroxy-1-(3-hydroxy-1-butinyl)-2,2,6-trimthylcyclohexylcarbonat wurden in 50 ml heissem Dimethylformamid gelöst. Die Lösung wurde mit 1 g Pyridinium-p-tosylat versetzt und 1,5 Stunden in einem vorgeheitzten Oelbad gerührt (Oelbadtemperatur 90-93 °C, Innentemperatur ca. 79-82 °C). Anschliessend wurde das Reaktionsgemisch auf 500 ml halbgesättigte Kochsalzlösung gegossen und dreimal mit je 200 ml Diäthyläther extrahiert. Die organischen Phasen wurden einmal mit gesättigter Kochsalzlösung gewaschen, über Natriumsulfat getrocknet und eingedampft. Das erhaltene Rohprodukt (8,3 g) wurde an Kieselgel mit Hexan/Diäthyläther (Vol. 1 : 1) chromatographiert. Hierbei wurden 5,9 g (83,3 %) (4R)-4-(4-Hydroxy-2,6,6-trimethyl-1-cyclohexenyl)-3-butin-2-ol erhalten.

Das als Ausgangsmaterial verwendete Aethyl-(1S,4R,6R)-4-hydroxy-1-(3-hydroxy-1-butinyl)-2,2,6-trimethylcyclohexylcarbonat wurde wie folgt hergestellt:

a) In einem Vierhalskolben mit Magnetrührer, Thermometer, Tropftrichter und einer Vorrichtung zur Inertbegasung wurden unter Argon 19,52 g (4R, 6R)-4-Hydroxy-2,2,6-trimethylcyclohexanon in einem Gemisch von 40 ml absolutem Tetrahydrofuran und 20 mg Pyridinium-p-tosylat gelöst und dann innert ca. 20 Minuten bei 15-20 °C 14,4 g Isopropenylmethyläther zur Lösung zugetropft. Die erhaltene Lösung A wurde gemäss Absatz c) weiterverwendet.

b) In einem Sulfierkolben mit Rührwerk, Thermometer, Tropftrichter, Steigrohr und einer Vorrichtung zur Inertbegasung wurde eine Lösung von 23,5 g 3-Butin-2-yl-trimethylsilyläther in 80 ml absolutem Tetrahydrofuran unter Argon vorgelegt auf — 30 °C gekühlt und innert 10 Minuten bei — 30 °C bis — 20 °C tropfenweise mit 105 ml einer ca. 1,56 M Lösung von Butyllithium in Hexan versetzt. Das Reaktionsgemisch wurde noch 30 Minuten bei — 10 °C gerührt und dann erneut auf — 40 °C abgekühlt. Die erhaltene Lösung B wurde gemäss Absatz c) weiterverwendet.

c) Lösung A wurde bei — 40 °C innert 10 Minuten zu Lösung B zugetropft. Das Gemisch wurde noch 30 Minuten bei — 40 °C nachgerührt, dann mit 15,7 ml Chlorameisensäureäthylester versetzt und unter Rühren innert 1 Stunde auf Raumtemperatur erwärmt. Anschliessend wurde das Reaktionsgemisch auf 0 °C gekühlt, unter Rühren mit 100 ml 3N Schwefelsäure versetzt und noch 30 Minuten bei 0-5 °C weiter gerührt. Das Reaktionsgemisch wurde mit 250 ml Aethylacetat verdünnt und von der wässrigen Phase abgetrennt. Die wässrige Phase wurde zweimal mit je 250 ml Aethylacetat nachextrahiert. Die organischen Phasen wurden dreimal mit je 250 ml gesättigter Natriumhydrogencarbonat-Lösung und einmal mit 250 ml gesättigter Kochsalzlösung gewaschen, vereinigt und über Natriumsulfat getrocknet. Nach Abfiltrieren des Trocknungsmittels und Einengen am Rotationsverdampfer (Wasserbadtemperatur 50 °C) wurden 43,6 g Rohprodukt erhalten, welches an Kieselgel mit Hexan/Diäthyläther (Vol. 1 : 1) chromatographiert wurde. Aus den produkthaltigen Fraktionen wurden insgesamt 36,5 g (97,8 %) Aethyl-(1S,4R,6R)-4-hydroxy-1-(3-hydroxy-1-butinyl)-2,2,6-trimethylcyclohexylcarbonat als schwach gelbliches Oel erhalten.

## Beispiel 2

In einem Zweihalskolben mit Magnetrührer, Thermometer, Rückflusskühler und Argonaufsatz wurden unter Argon 6,7 g Imidazol in 40 ml Triäthylenglykoldimethyläther gelöst und die Lösung im Oelbad auf 195 °C erwärmt. Anschliessend wurde bei einer Innentemperatur von 188-190 °C innert 60 Minuten eine Lösung von 8,0 g Aethyl-(1S,4R,6R)-4-hydroxy-1-(5-hydroxy-3-methyl-3E-penten-1-inyl)-2,2,6-trimethylcyclohexylcarbonat in 15 ml Triäthylenglykoldimethyläther zugetropft. Das Reaktionsgemisch wurde noch 30 Minuten bei gleicher Temperatur gehalten und dann auf 200 ml Eis/Wasser gegossen.

Die wässrige Phase wurde zweimal mit je 250 ml Diäthyläther extrahiert. Die vereinigten Aether-Phasen wurden viermal mit je 100 ml Wasser gewaschen, über Natriumsulfat getrocknet, am Rotationsverdampfer bei ca. 50 °C eingeengt und dann 3 Stunden im Hochvakuum bei Raumtemperatur getrocknet. Das erhaltene Rohprodukt (6,0 g) wurde an Kieselgel mit Diäthyläther/Hexan (Vol. 1 : 1) chromatographiert, wobei 3,5 g (61,4 %) (4R)-5-(4-Hydroxy-2,6,6-trimethyl-1-cyclohexenyl)-3-methyl-2-penten-4-in-1-ol als leicht gelbliches Oel isoliert werden konnten (Verhältnis 2E/2Z = 91,0 : 4,7).

Das als Ausgangsmaterial verwendet Aethyl-(1S,4R,6R)-4-hydroxy-1-(5-hydroxy-3-methyl-3E-penten-1-inyl)-2,2,6-trimethylcyclohexylcarbonat wurde wie folgt hergestellt :

a) In einem Vierhalskolbem mit Magnetrührer, Thermometer, Tropftrichter und einer Vorrichtung zur Inertbegasung wurden unter Argon 52,0 g (4R,6R)-4-Hydroxy-2,2,6-trimethylcyclohexanon in 65 ml Tetrahydrofuran und 50 mg Pyridinium-p-tosylat gelöst und die Lösung bei 15-20 °C innert 20 Minuten tropfenweise mit 48,0 g Isopropenylmethyläther versetzt. Die erhaltene gelbliche Lösung A wurde gemäss Absatz c) direkt weiterverwendet.

b) In einem Sulfierkolben mit Rührwerk, Thermometer, Tropftrichter, Steigrohr und einer Vorrichtung zur Inertbegasung wurde eine Lösung von 79 g 5-(2-Methoxy-2-propyl)oxy-3-methyl-3E-penten-1-in in 50 ml absolutem Tetrahydrofuran unter Argon vorgelegt und auf — 20 °C gekühlt. Diese Lösung wurde nun bei — 30 °C bis — 20 °C innert 20 Minuten tropfenweise mit 280 ml einer ca. 1,56 M Lösung von Butyllithium in Hexan versetzt und noch 30 Minuten nachgerührt. Die erhaltene orange Lösung B wurde gemäss Absatz c) weiterverwendet.

c) Lösung B wurde bei — 15 °C innert 20 Minuten tropfenweise mit Lösung A versetzt und das Gemisch noch 2 Stunden bei Raumtemperatur nachgerührt. Das Reaktionsgemisch wurde auf — 20 °C gekühlt, unter Rühren mit 42 ml Chlorameisensäureäthylester versetzt und dann während einer Stunde auf Raumtemperatur erwärmt. Anschliessend wurde das Reaktionsgemisch mit 250 ml Diäthyläther verdünnt und die wässrige Phase abgetrennt und mit 250 ml Diäthyläther nachextrahiert. Die organischen Phasen wurden zweimal mit je 250 ml gesättigter Kochsalzlösung gewaschen, vereinigt und über Natriumsulfat getrocknet. Nach Abfiltrieren des Trocknungsmittels und Einengen am Rotationsverdampfer (Wasserbadtemperatur 50 °C) wurden 181,5 g rohes Aethyl-(1S,4R,6R)-4-(2-methoxy-2-propyl)oxy-1-[5-(2-methoxy-2-propyl)oxy-3-methyl-3E-penten-1-inyl]-2,2,6-triméthylcyclohexylcarbonat als braungelbes Oel erhalten.

d) Das erhaltene braungelbe Oel wurde in 450 ml Tetrahydrofuran und 50 ml Wasser gelöst (trübe Lösung), mit 2 g Pyridinium-p-tosylat versetzt und noch 20 Minuten nachgerührt (klare Lösung). Anschliessend wurde die braunorange Reaktionslösung mit 2 g festem Kaliumcarbonat versetzt und am Rotationsverdampfer (Badtemperatur 50 °C) bis zu konstantem Gewicht eingedampft. Der Rückstand

wurde mit 250 ml Diäthyläther verdünnt und mit 250 ml Wasser extrahiert. Die wässrige Phase wurde abgetrennt und mit 250 ml Diäthyläther nachextrahiert. Die organischen Phasen wurden zweimal mit je 250 ml gesättigter Kochsalzlösung gewaschen, vereinigt und über Natriumsulfat getrocknet. Nach. Abfiltrieren des Trocknungsmittels und Einengen des Filtrates am Rotationsverdampfer (Wasserbadtemperatur 50 °C) wurden, 140 g Rohprodukt erhalten, welches an Kieselgel mit Diäthyläther/Hexan (Vol. 1 : 1) chromatographiert wurde. Aus den produkthaltigen Fraktionen wurden insgesamt 99,0 g (91,6 %) reines Aethyl-(1S,4R,6R)-4-hydroxy-1-(5-hydroxy-3-methyl-3E-penten-1-inyl)-2,2,6-trimethylcyclohexylcarbonat als leicht gelbes Oel erhalten.

## Beispiel 3

In einem Zweihalskolben mit Magnetrührer, Rückflusskühler und Argonaufsatz wurden unter Argon 4,4 g Imidazol in 50 ml Triäthylenglykoldimethyläther gelöst und die Lösung im Oelbad auf 195 °C erwärmt. Anschliessend wurde bei einer Innentemperatur von 185-190 °C innert ca. 20 Minuten eine Lösung von 5 g Aethyl-1-(5-hydroxy-3-methyl-3E-penten-1-inyl)-2,2,6-trimethylcyclohexylcarbonat in 10 ml Triäthylenglykol-dimethyläther zugetropft. Das Reaktionsgemisch wurde noch 1 Stunde bei gleicher Temperatur gehalten und dann auf 200 ml Eis/Wasser gegossen. Die wässrige Phase wurde zweimal mit je 250 ml Diäthyläther extrahiert. Die vereinigten Aether-Phasen wurden viermal mit je 100 ml Wasser gewaschen, über Natriumsulfat getrocknet, am Rotationsverdampfer bei ca. 40 °C eingeengt und dann 2 Stunden im Hochvakuum bei Raumtemperatur getrocknet. Das erhaltene Rohprodukt (3,6 g) wurde an Kieselgel mit Diäthyläther/Hexan (Vol. 1 : 1) chromatographiert. Aus den produkthaltigen Fraktionen wurden 2,1 g (59,5 %) 5-(2,6,6-Trimethyl-1-cyclohexenyl)-3-methyl-2-penten-4-in-1-ol als leicht gelbes Oel erhalten (Verhältnis 2E/2Z = 98,8 : 0,6).

Das als Ausgangsmaterial verwendete Aethyl-1-(5-hydroxy-3-methyl-3E-penten-1-inyl)-2,2,6-trimethylcyclohexylcarbonat wurde wie folgt hergestellt :

a) In einem Sulfierkolben mit Rührer, Thermometer, Tropftrichter und Argonaufsatz wurden 115,1 g 5-(2-Methoxy-2-propyl)oxy-3-methyl-3E-penten-1-in und 90 ml Tetrahydrofuran vorgelegt, auf — 25 °C abgekühlt und bei dieser Temperatur innert 20 Minuten tropfenweise mit einer 1,6 M Lösung von Butyllithium in Hexan versetzt. Das Gemisch wurde noch 15 Minuten bei 0 °C nachgerührt, dann auf — 10 °C abgekühlt und bei dieser Temperatur innert 15 Minuten tropfenweise mit 72,9 g 2,2,6-Trimethylcyclohexanon versetzt. Anschliessend wurde das Reaktionsgemisch auf Raumtemperatur erwärmt und noch 2 Stunden nachgerührt. Das Gemisch wurde auf 0 °C abgekühlt, bei dieser Temperatur innert 15 Minuten tropfenweise mit 67,5 ml Chlorameisensäureäthylester versetzt, auf Raumtemperatur erwärmen gelassen und noch 1,5 Stunden nachgerührt. Anschliessend wurde das Reaktionsgemisch auf 1 l gesättigte Natriumhydrogencarbonat-Lösung gegossen und zweimal mit je 1 l Diäthyläther extrahiert. Die organischen Phasen wurden mit 1 l entionisiertem Wasser gewaschen, über Natriumsulfat getrocknet, filtriert und am Rotationsverdampfer im Vakuum eingeengt. (Bad-temperatur ca. 45 °C). Der Rückstand wurde am Hochvakuum bei 60 °C Badtemperatur während 1 Stunde vom 5-(2-Methoxy-2-propyl) oxy-3-methyl-3E-penten-1-in befreit. Es wurden 232,1 g (117,3 %) rohes Aethyl-1-[5-(2-methoxy-2-propyl) oxy-3-methyl-3E-penten-1-inyl]-2,2,6-trimethylcyclohexylcarbonat erhalten, welches ohne weitere Reinigung direkt weiterverwendet wurde.

b) In einem Sulfierkolben mit Rührer, Thermometer und Argonaufsatz wurden 105,0 g des gemäss Absatz a) erhaltenen Rohproduktes in 630 ml Tetrahydrofuran gelöst, mit 105 ml entionisiertem Wasser und 5,25 g Pyridinium-p-tosylat versetzt und noch 30 Minuten bei Raumtemperatur nachgerührt. Das Reaktionsgemisch wurde auf 750 ml gesättigter Natriumhydrogencarbonat-Lösung gegossen und dreimal mit je 600 ml Diäthyläther extrahiert. Die organischen Phasen wurden mit 750 ml halbgesättigter Natirumhydrogencarbonat-Lösung gewaschen, über Natriumsulfat getrocknet, filtriert und am Rotationsverdampfer im Vakuum eingeengt (Badtemperatur ca. 45 °C). Nach dem Trocknen am Hochvakuum wurden 84,8 g (116,9 %) Rohprodukt erhalten, welches an Kieselgel mit Hexan/Diäthyl-äther (Vol. 2 : 1) chromatographiert wurde. Hierbei wurden 2,3 g (4,5 %) 5-(2,6,6-Trimethyl-1-cyclohexenyl)-3-methyl-2-penten-4-in-1-ol und 53,6 g (73,9 %) Aethyl-1-(5-hydroxy-3-methyl-3E-penten-1-inyl)-2,2,6-trimethylcyclohexylcarbonat erhalten.

## Beispiel 4

In einem Sulfierkolben mit Rührer, Thermometer, Tropftrichter, Rückflusskühler und einer Vorrichtung zur Inertbegasung wurden 16 ml tert.-Butanol und 20 ml absolutes Toluol unter Argon vorgelegt und auf — 25 °C gekühlt. Diese Lösung wurde bei — 30 °C bis — 20 °C innert 10 Minuten tropfenweise mit 52 ml einer 1,56 M Lösung von Butyllithium in Hexan versetzt und noch 30 Minuten ohne Kühlung nachgerührt. Anschliessend wurde bei ca. 20 °C zu der so hergestellten Lösung von Lithium-tert.-butylat eine Lösung von 14,4 g (1S,4R,6R)-4-Hydroxy-1-(5-hydroxy-3-methyl-3E-penten-1-inyl)-2,2,6-trimethylcyclohexylbenzoat in 120 ml absolutem Toluol auf einmal zugegeben. Die erhaltene Suspension wurde mit Hilfe eines Oelbades vorsichtig auf 65 °C erwärmt und 1,5 Stunden bei dieser Temperatur nachgerührt.

Danach wurde das Reaktionsgemisch auf 150 ml Eis/Wasser gegossen. Die wässrige Phase wurde abgetrennt und zweimal mit je 150 ml Diäthyläther extrahiert. Die organischen Phasen wurde mit 150 ml gesättigter Kochsalzlösung gewaschen, vereinigt, über Natriumsulfat getrocknet, am Rotationsverdampfer bei ca. 40 °C eingeengt und dann 1 Stunde im Hochvakuum bei Raumtemperatur getrocknet. Das erhaltene Rohprodukt (12,0 g) wurde an Kieselgel mit Diäthyläther/Hexan (Vol. 1 : 1) chromatographiert. Aus den produkthaltigen Fraktionen wurden insgesamt 7,5 g (80 %) (4R)-5-(4-Hydroxy-2,6,6-trimethyl-1-cyclohexen-1-yl)-3-methyl-2E-penten-4-in-1-ol als gelbliche Kristalle erhalten (Reinheit 98,8 %, Gehalt an 2Z-Isomer 1,1 %).

Das als Ausgangsmaterial verwendete (1S,4R,6R)-4-Hydroxy-1-(5-hydroxy-3-methyl-3E-penten-1-in-yl)-2,2,6-trimethylcyclohexylbenzoat wurde wie folgt hergestellt :

a) Eine Lösung von 16,4 g (4R,6R)-4-Hydroxy-2,2,6-trimethylcyclohexanon und 30 mg Pyridinium-p-tosylat in 20 ml Tetrahydrofuran wurden unter Argon bei 15-20 °C innert 10 Minuten tropfenweise mit 16,0 g Isopropenylmethyläther versetzt.

b) Eine Lösung von 27 g 5-(2-Methoxy-2-propyl)oxy-3-methyl-3E-penten-1-in in 30 ml absolutem Tetrahydrofuran wurde unter Argon auf — 20 °C gekühlt, dann bei — 30 °C bis — 20 °C innert 10 Minuten tropfenweise mit 90 ml einer ca. 1,56 M Lösung von Butyllithium in Hexan versetzt und noch 30 Minuten nachgerührt.

c) Die gemäss Absatz b) erhaltene Lösung wurde bei — 15 °C innert 15 Minuten tropfenweise mit der gemäss Absatz a) hergestellten Lösung versetzt und das Gemisch noch 1 Stunde bei Raumtemperatur nachgerührt. Das Reaktionsgemisch wurde auf — 10 °C gekühlt, unter Rühren mit 18,6 ml Benzoylchlorid versetzt und dann über Nach (18 Stunden) bis Raumtemperatur nachgerührt. Danach wurde das Reaktionsgemisch auf 200 ml gesättigte Natriumhydrogencarbonat-Lösung gegossen, die wässrige Phase abgetrennt und zweimal mit je 300 ml Diäthyläther nachextrahiert. Die organischen Phasen wurden zweimal mit je 250 ml gesättigter Kochsalzlösung gewaschen, vereinigt, über Natriumsulfat getrocknet und filtriert. Nach Einengen des Filtrates am Rotationsverdampfer (Wasserbadtemperatur 40 °C) wurde rohes (1S,4R,6R)-4-(2-Methoxy-2-propyl)oxy-1-[5-(2-methoxy-2-propyl)oxy-3-methyl-3E-penten-1-inyl]-2,2,6-trimethylcyclohexylbenzoat als braungelbes Oel erhalten.

d) Das erhaltene braungelbe Oel wurde in 400 ml Tetrahydrofuran und 20 ml Wasser gelöst (trübe Lösung), mit 1 g Pyridinium-p-tosylat versetzt und noch 15 Minuten nachgerührt (klare Lösung). Anschliessend wurde das Reaktionsgemisch auf 200 ml gesättigte Natriumhydrogencarbonat-Lösung gegossen, die wässrige Phase abgetrennt und zweimal mit je 300 ml Diäthyläther extrahiert. Die organischen Phasen wurden vereinigt, über Natriumsulfat getrocknet und filtriert. Nach Einengen des Filtrates am Rotationsverdampfer (Wasserbadtemperatur 40 °C) wurden 51,3 Rohprodukt erhalten, welches an Kieselgel mit Diäthyläther/Hexan (Vol. 2 : 1) chromatographiert wurde. Aus den produkthaltigen Fraktionen wurden insgesamt 32,2 g (86,1 %) reines (1S, 4R, 6R)-4-Hydroxy-1-(5-hydroxy-3-methyl-3E-penten-1-inyl)-2,2,6-trimethylcyclohexylbenzoat als leicht gelbes Oel erhalten.

Beispiel 5

In einem Sulfierkolben mir Rührer, Thermometer, Tropftrichter, Rückflusskühler und einer Vorrichtung zur Inertbegasung wurden 40 ml tert.-Butanol und 50 ml absolutes Toluol unter Argon vorgelegt und auf — 25 °C gekühlt. Diese Lösung wurde bei —30 °C bis — 20 °C innert 10 Minuten tropfenweise mit 130 ml einer 1,56 M Lösung von Butyllithium in Hexan versetzt und noch 30 Minuten ohne Kühlung nachgerührt. Anschliessend wurde bei 6 °C zu der so hergestellten Lösung von Lithium-tert.-butylat eine Lösung von 32,4 g Aethyl-(1S,4R,6R)-4-hydroxy-1-(5-hydroxy-3-methyl-3E-penten-1-inyl)-2,2,6-trimethylcyclohexylcarbonat (hergestellt nach Beispiel 2) in 150 ml absolutem Toluol auf einmal zugegeben. Das Reaktionsgemisch wurde mit Hilfe eines Oelbades vorsichtig auf 55 °C erwärmt und 30 Minuten bei dieser Temperatur nachgerührt. Danach wurde das Reaktionsgemisch auf 400 ml Eis/Wasser gegossen. Die wässrige Phase wurde abgetrennt und zweimal mit je 300 ml Diäthyläther extrahiert. Die organischen Phasen wurden mit 300 ml gesättigter Kochsalzlösung gewaschen, vereinigt, über Natriumsulfat getrocknet, am Rotationsverdampfer bei ca. 40 °C eingeengt und dann 5 Stunden im Hochvakuum bei Raumtemperatur getrocknet. Hierbei wurden 23,9 g (102,1 %) gelb-oranges, kristallines Rohprodukt von (4R)-5-(4-Hydroxy-2,6,6-trimethyl-1-cyclohexenyl)-3-methyl-2-penten-4-in-1-ol (enthaltend 90,5 % 2E-Isomer mit 1,4 % 2Z-Isomer) erhalten, welches an Kieselgel mit Diäthyläther/Hexan (Vol. 1 : 1) chromatographiert wurde. Aus den produkthaltigen Fraktionen wurden insgesamt 19,9 g (85 %) (4R)-5-(4-Hydroxy-2,6,6-trimethyl-1-cyclohexen-1-yl)-3-methyl-2E-penten-4-in-1-ol als gelbliche Kristalle erhalten (Reinheit 98,0 %, Gehalt an 2Z-Isomer 0,5 %).

**Patentansprüche** (für die Vertragsstaaten : BE, CH, DE, FR, GB, IT, LI, NL)

1. Verfahren zur Herstellung von Cycloalkenylalkinen, dadurch gekennzeichnet, dass man eine

Verbindung der allgemeinen Formel

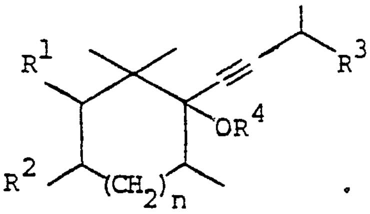

(I)

worin n für die Zahl 0 oder 1 steht ; einer der Reste $R^1$ und $R^2$ Wasserstoff und der andere Wasserstoff, eine Hydroxy- oder Oxogruppe oder eine geschützte Hydroxy- oder Oxogruppe bezeichnet ; $R^3$ Hydroxy, die Oxogruppe, $=CH—CH_2—OH$ oder =CH-CHO oder eine von diesen Gruppen abgeleitete Gruppe mit geschützter Hydroxy- oder Oxofunktion bedeutet ;. $R^4$ $—CO—OR^6$, $—CO—R^6$, $—CO—NR^6R^7$, —CO—Cl oder $—SO_2—R^6$ darstellt ; und $R^6$ und $R^7$ gesättigte oder aromatische Kohlenwasserstoffreste oder $R^7$ auch Wasserstoff bedeuten, durch Abspaltung von $R^4OH$ in eine Verbindung der allgemeinen Formel

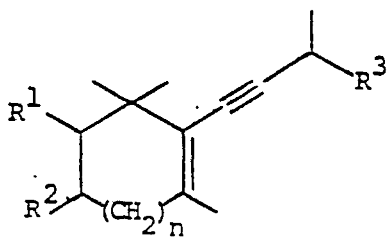

(II)

worin n, $R^1$, $R^2$ und $R^3$ die obigen Bedeutungen haben, überführt und, gewünschtenfalls, eine Verbindung der Formel II, worin $R^1$, $R^2$ oder $R^3$ eine geschützte Hydroxy- oder Oxo-gruppe aufweist, zur entsprechenden Hydroxy- oder Oxoverbindung hydrolysiert.

2. Verfahren nach Anspruch 1, dadurch gekennzeichnet, dass die Abspaltung von $R^4OH$ mittels Erhitzen erfolgt.

3. Verfahren nach Anspruch 1 oder 2, dadurch gekennzeichnet, dass die Umsetzung der Verbindung der Formel I in Gegenwart eines Katalysators erfolgt.

4. Verfahren nach einem der Ansprüche 1 bis 3, dadurch gekennzeichnet, dass die Umsetzung in Gegenwart einer organischen Stickstoffbase oder eines Salzes solcher Basen oder eines Lithiumsalzes, vorzugsweise in Gegenwart von Imidazol, 1,2,4-Triazol, Anilin, Pyridinium-p-tosylat, Lithiumchlorid, Lithiumperchlorat, Lithium-tert.-butylat oder Lithiumtetrafluoroborat erfolgt.

5. Verfahren nach einem der Ansprüche 1 bis 4, dadurch gekennzeichnet, dass man eine Verbindung der Formel I umsetzt, worin n für die Zahl 1 steht und $R^1$ Wasserstoff bedeutet.

6. Verfahren nach einem der Ansprüche 1 bis 5, dadurch gekennzeichnet, dass man eine Verbindung der Formel I umsetzt, worin einer der Reste $R^1$ und $R^2$ Wasserstoff und der andere Wasserstoff oder Hydroxy, vorzugsweise Hydroxy bezeichnet.

7. Verfahren nach einem der Ansprüche 1 bis 6, dadurch gekennzeichnet, dass man eine Verbindung der Formel I umsetzt, worin $R^3$ Hydroxy oder die Gruppe $=CH—CH_2—OH$ bedeutet.

8. Verfahren nach einem der Ansprüche 1 bis 7, dadurch gekennzeichnet, dass man eine Verbindung der allgemeinen Formel

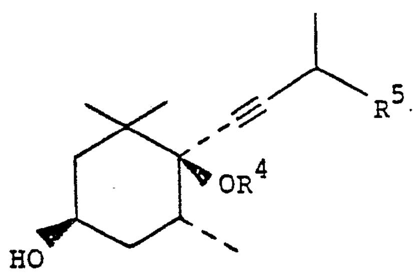

(Ia)

worin $R^5$ Hydroxy oder die Gruppe $=CH—CH_2—OH$ bezeichnet und $R^4$ die in Anspruch 1 gegebene Bedeutung hat, oder deren optischen Antipoden in Gegenwart einer basischen, sterisch gehinderten Lithiumverbindung umsetzt.

9. Verfahren nach einem der Ansprüche 1 bis 8, dadurch gekennzeichnet, dass man eine Verbindung der Formel I bzw. Ia umsetzt, worin $R^4$ einen Rest der Formel $—CO—OR^6$ oder $—CO—R^6$, vorzugsweise $—CO—OCH_3$, $—CO—OC_2H_5$ oder $—CO—C_6H_5$ darstellt.

10. Verbindungen der allgemeinen Formel

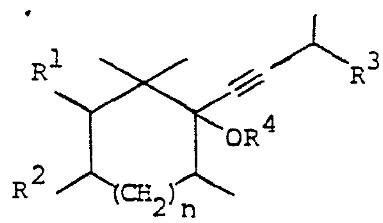

worin n für die Zahl 0 oder 1 steht ; einer der Reste $R^1$ und $R^2$ Wasserstoff und der andere Wasserstoff, eine Hydroxy- oder Oxogruppe oder eine geschützte Hydroxy- oder Oxogruppe bezeichnet ; $R^3$ Hydroxy, die Oxogruppe, $=CH-CH_2-OH$ oder $=CH-CHO$ oder eine von diesen Gruppen abgeleitete Gruppe mit geschützter Hydroxy- oder Oxofunktion bedeutet ; $R^4$ $-CO-OR^6$, $-CO-R^6$, $-CO-NR^6R^7$, $-CO-Cl$ oder $-SO_2-R^6$ darstellt ; und $R^6$ und $R^7$ gesättigte oder aromatische Kohlenwasserstoffreste oder $R^7$ auch Wasserstoff bedeuten.

**Patentansprüche** (für den Vertragsstaat AT)

1. Verfahren zur Herstellung von Cycloalkenylalkinen dadurch gekennzeichnet, dass man eine Verbindung der allgemeinen Formel

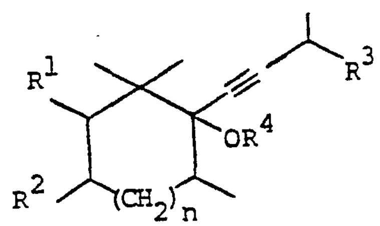

(I)

worin n für die Zahl 0 oder 1 steht ; einer der Reste $R^1$ und $R^2$ Wasserstoff und der andere Wasserstoff, eine Hydroxy- oder Oxogruppe oder eine geschützte Hydroxy- oder Oxogruppe bezeichnet ; $R^3$ Hydroxy, die Oxogruppe, $=CH-CH_2-OH$ oder $=CH-CHO$ oder eine von diesen Gruppen abgeleitete Gruppe mit geschützter Hydroxy- oder Oxofunktion bedeutet ; $R^4$ $-CO-OR^6$, $-CO-R^6$, $-CO-NR^6R^7$, $-CO-Cl$ oder $-SO_2-R^6$ darstellt ; und $R^6$ und $R^7$ gesättigte oder aromatische Kohlenwasserstoffreste oder $R^7$ auch Wasserstoff bedeuten, durch Abspaltung von $R^4OH$ in eine Verbindung der allgemeinen Formel

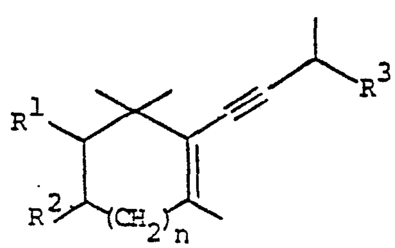

(II)

worin n, $R^1$, $R^2$ und $R^3$ die obigen Bedeutungen haben, überführt und, gewünschtenfalls, eine Verbindung der Formel II, worin $R^1$, $R^2$ oder $R^3$ eine geschützte Hydroxy- oder Oxogruppe aufweist, zur entsprechenden Hydroxy- oder Oxoverbindung hydrolysiert.

2. Verfahren nach Anspruch 1, dadurch gekennzeichnet, dass die Abspaltung von $R^4OH$ mittels Erhitzen erfolgt.

3. Verfahren nach Anspruch 1 oder 2, dadurch gekennzeichnet, dass die Umsetzung der Verbindung der Formel I in Gegenwart eines Katalysators erfolgt.

4. Verfahren nach einem der Ansprüche 1 bis 3, dadurch gekennzeichnet, dass die Umsetzung in Gegenwart einer organischen Stickstoffbase oder eines Salzes solcher Basen oder eines Lithiumsalzes, vorzugsweise in Gegenwart von Imidazol, 1,2,4-Triazol, Anilin, Pyridinium-p-tosylat, Lithiumchlorid, Lithiumperchlorat, Lithium-tert.-butylat oder Lithiumtetrafluoroborat erfolgt.

5. Verfahren nach einem der Ansprüche 1 bis 4, dadurch gekennzeichnet, dass man eine Verbindung der Formel I umsetzt, worin n für die Zahl 1 steht und $R^1$ Wasserstoff bedeutet.

6. Verfahren nach einem der Ansprüche 1 bis 5, dadurch gekennzeichnet, dass man eine Verbindung der Formel I umsetzt, worin einer der Reste $R^1$ und $R^2$ Wasserstoff und der andere Wasserstoff oder Hydroxy, vorzugsweise Hydroxy bezeichnet.

7. Verfahren nach einem der Ansprüche 1 bis 6, dadurch gekennzeichnet, dass man eine Verbindung der Formel I umsetzt, worin $R^3$ Hydroxy oder die Gruppe $=CH-CH_2-OH$ bedeutet.

8. Verfahren nach einem der Ansprüche 1 bis 7, dadurch gekennzeichnet, dass man eine Verbindung der allgemeinen Formel

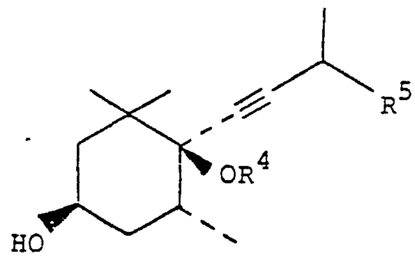

(Ia)

worin $R^5$ Hydroxy oder die Gruppe $=CH—CH_2—OH$ bezeichnet und $R^4$ die in Anspruch 1 gegebene Bedeutung hat, oder deren optischen Antipoden in Gegenwart einer basischen, sterisch gehinderten Lithiumverbindung umsetzt.

9. Verfahren nach einem der Ansprüche 1 bis 8, dadurch gekennzeichnet, dass man eine Verbindung der Formel I bzw. Ia umsetzt, worin $R^4$ einen Rest der Formel $—CO—OR^6$ oder $—CO—R^6$, vorzugsweise $—CO—OCH_3$, $—CO—OC_2H_5$ oder $—CO—C_6H_5$ darstellt.

**Claims** (for the Contracting States : BE, CH, DE, FR, GB, IT, LI, NL)

1. A process for the manufacture of cycloalkenylalkynes, characterized by converting a compound of the general formula

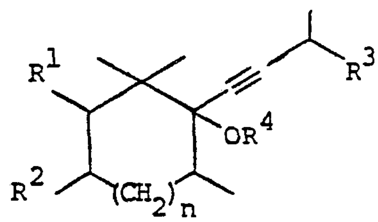

(I)

wherein n stands for the number 0 or 1 ; one of the residues $R^1$ and $R^2$ denotes hydrogen and the other denotes hydrogen, a hydroxy or oxo group or a protected hydroxy or oxo group ; $R^3$ signifies hydroxy, the oxo group, $=CH—CH_2—OH$ or $=CH—CHO$ or a group derived from these groups with a protected hydroxy or oxo function ; $R^4$ represents $—OO—OR^6$; $—CO—R^6$, $—CO—NR^6R^7$, $—CO—Cl$ or $—SO_2—R^6$ ; and $R^6$ and $R^7$ signify saturated or aromatic hydrocarbon residues or $R^7$ also signifies hydrogen, by cleavage of $R^4OH$ into a compound of the general formula

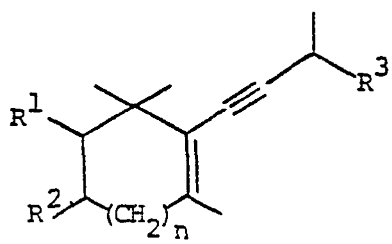

(II)

wherein n, $R^1$, $R^2$ and $R^3$ have the above significances, and, if desired, hydrolyzing a compound of formula II in which $R^1$, $R^2$ or $R^3$ carries a protected hydroxy or oxo group to the corresponding hydroxy or oxo compound.

2. A process according to claim 1, characterized in that the cleavage of $R^4OH$ is carried out by heating.

3. A process according to claim 1 or 2, characterized in that the reaction of the compound of formula I is carried out in the presence of a catalyst.

4. A process according to any one of claims 1 to 3, characterized in that the reaction is carried out in the presence of an organic nitrogen base or a salt of such a base or a lithium salt, preferably in the presence of imidazole, 1,2,4-triazole, aniline, pyridinium p-tosylate, lithium chloride, lithium perchlorate, lithium tert.-buytylate or lithium tetrafluoroborate.

5. A process according to any one of claims 1 to 4, characterized in that a compound of formula I in which n stands for the number 1 and $R^1$ signifies hydrogen is reacted.

6. A process according to any one of claims 1 to 5, characterized in that a compound of formula I in which one of the residues $R^1$ and $R^2$ denotes hydrogen and the other denotes hydrogen or hydroxy, preferably hydroxy, is reacted.

7. A process according to any one of claims 1 to 6, characterized in that a compound of formula I in

which $R^3$ signifies hydroxy or the group $=CH—CH_2—OH$ is reacted.

8. A process according to any one of claims 1 to 7, characterized in that a compound of the general formula

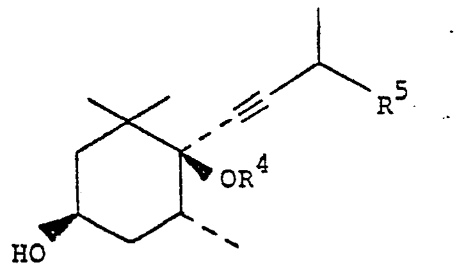

(Ia)

wherein $R^5$ denotes hydroxy or the group $=CH—CH_2—OH$ and $R^4$ has the significance given in claim 1, or its optical antipode is reacted in the presence of a basic, sterically hindered lithium compound.

9. A process according to any one of claims 1 to 8, characterized in that a compound of formula I or Ia in which $R^4$ represents a residue of the formula $—CO—OR^6$ or $—CO—R^6$, preferably $—CO—OCH_3$, $—CO—OC_2H_5$ or $—CO—C_6H_5$, is reacted.

10. Compounds of the general formula

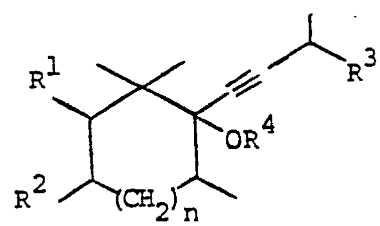

wherein n stands for the number 0 or 1 ; one of the residues $R^1$ and $R^2$ denotes hydrogen and the other denotes hydrogen, a hydroxy or oxo group or a protected hydroxy or oxo group ; $R^3$ signifies hydroxy, the oxo group, $=CH—CH_2—OH$ or $=CH—CHO$ or a group derived from these groups with a protected hydroxy or oxo function ; $R^4$ represents $—CO—OR^6$, $—CO—R^6$, $—CO—NR^6R^7$, $—CO—Cl$ or $—SO_2—R^6$ ; and $R^6$ and $R^7$ signify saturated or aromatic hydrocarbon residues or $R^7$ also signifies hydrogen.

**Claims** (for the Contracting State AT)

1. A process for the manufacture of cycloalkenylalkynes, characterized by converting a compound of the general formula

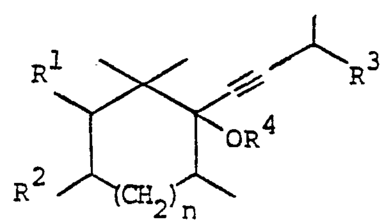

(I)

wherein n stands for the number 0 or 1 ; one of the residues $R^1$ and $R^2$ denotes hydrogen and the other denotes hydrogen, a hydroxy or oxo group or a protected hydroxy or oxo group ; $R^3$ signifies hydroxy, the oxo group, $=CH—CH_2—OH$ or $=CH—CHO$ or a group derived from these groups with a protected hydroxy or oxo function ; $R^4$ represents $—CO—OR^6$, $—CO—R^6$, $—CO—NR^6R^7$, $—CO—Cl$ or $—SO_2—R^6$ ; and $R^6$ and $R^7$ signify saturated or aromatic hydrocarbon residues or $R^7$ also signifies hydrogen, by cleavage of $R^4OH$ into a compound of the general formula

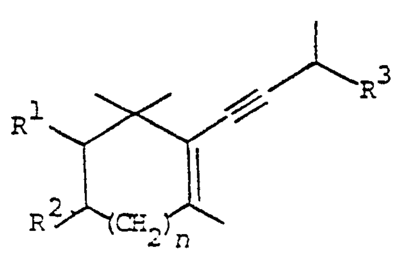

(II)

wherein n, $R^1$, $R^2$ and $R^3$ have the above significances, and if desired, hydrolizing a compound of formula II in which $R^1$, $R^2$ or $R^3$ carries a protected hydroxy or oxo group to the corresponding hydroxy or oxo compound.

2. A process according to claim 1, characterized in that the cleavage of $R^4OH$ is carried out by heating.

3. A process according to claim 1 or 2, characterized in that the reaction of the compound of formula I is carried out in the presence of a catalyst.

4. A process according to any one of claims 1 to 3, characterized in that the reaction is carried out in the presence of an organic nitrogen base or a salt of such a base or a lithium salt, preferably in the presence of imidazole, 1,2,4-triazole, aniline, pyridinium p-tosylate, lithium chloride, lithium perchlorate, lithium tert.-butylate or lithium tetrafluoroborate.

5. A process according to any one of claims 1 to 4, characterized in that a compound of formula I in which n stands for the number 1 and $R^1$ signifies hydrogen is reacted.

6. A process according to any one of claims 1 to 5, characterized in that a compound of formula I in which one of the residues $R^1$ and $R^2$ denotes hydrogen and the other denotes hydrogen or hydroxy, preferably hydroxy, is reacted.

7. A process according to any one of claims 1 to 6, characterized in that a compound of formula I in which $R^3$ signifies hydroxy or the group $=CH-CH_2-OH$ is reacted.

8. A process according to any one of claims 1 to 7, characterized in that a compound of the general formula

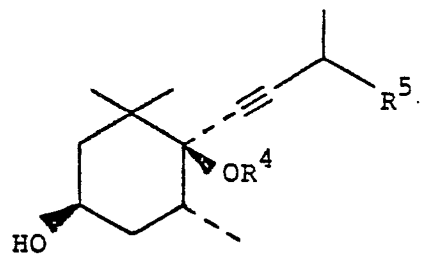

(Ia)

wherein $R^5$ denotes hydroxy or the group $=CH-CH_2-OH$ and $R^4$ has the significance given in claim 1, or its optical antipode is reacted in the presence of a basic, sterically hindered lithium compound.

9. A process according to any one of claims 1 to 8, characterized in that a compound of formula I or Ia in which $R^4$ represents a residue of the formula $-CO-OR^6$ or $-CO-R^6$, preferably $-CO-OCH_3$, $-CO-OC_2H_5$ or $-CO-C_6H_5$, is reacted.

**Revendications** (pour les Etats contractants : BE, CH, DE, FR, GB, IT, LI, NL)

1. Procédé pour la préparation de cycloalcénylalcynes, caractérisé en ce que l'on convertit un composé de formule générale

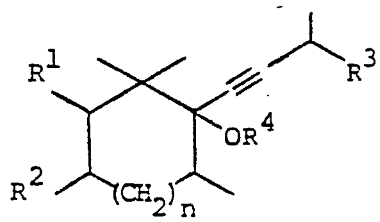

(I)

dans laquelle n est égal à 0 ou 1, l'un des symboles $R^1$ et $R^2$ représente l'hydrogène et l'autre l'hydrogène, un groupe hydroxy ou oxo ou un groupe hydroxy ou oxo protégé ; $R^3$ représente un groupe hydroxy, le groupe oxo, un groupe $=CH-CH_2-OH$ ou $=CH-CHO$ ou un groupe dérivé de ces derniers, à fonction hydroxy ou oxo protégée ; $R^4$ représente $-CO-OR^6$, $-CO-R^6$, $-CO-NR^6R^7$, $-CO-Cl$ ou $-SO_2-R^6$ ; et $R^6$ et $R^7$ représentent des radicaux hydrocarbonés saturés ou aromatiques, $R^7$ pouvant également représenter l'hydrogène, par scission de $R^4OH$, en un composé de formule générale

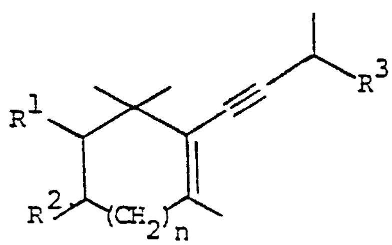

(II)

dans laquelle n, $R^1$, $R^2$ et $R^3$ ont les significations indiquées ci-dessus, et si on le désire, on hydrolyse un composé de formule II dans laquelle $R^1$, $R^2$ ou $R^3$ représente ou contient un groupe hydroxy ou oxo protégé, en le composé correspondant à groupe hydroxy ou oxo libre.

2. Procédé selon la revendication 1, caractérisé en ce que la scission de $R^4OH$ est provoquée par chauffage.

3. Procédé selon la revendication 1 ou 2, caractérisé en ce que la conversion du composé de formule I est réalisée en présence d'un catalyseur.

4. Procédé selon l'une des revendications 1 à 3, caractérisé en ce que la conversion est effectuée en présence d'une base organique azotée ou d'un sel d'une telle base ou d'un sel de lithium, de préférence en présence de l'imidazole, du 1,2,4-triazole, de l'aniline, du p-toluène-sulfonate de pyridinium, du chlorure de lithium, du perchlorate de lithium, du tert-butylate de lithium ou du tétrafluoborate de lithium.

5. Procédé selon l'une des revendications 1 à 4, caractérisé en ce que l'on convertit un composé de formule I dans laquelle n est égal à 1 et $R^1$ représente l'hydrogène.

6. Procédé selon l'une des revendications 1 à 5, caractérisé en ce que l'on convertit un composé de formule I dans laquelle l'un des symboles $R^1$ et $R^2$ représente l'hydrogène et l'autre l'hydrogène ou un groupe hydroxy, de préférence un groupe hydroxy.

7. Procédé selon l'une des revendications 1 à 6, caractérisé en ce que l'on convertit un composé de formule I dans laquelle $R^3$ représente un groupe hydroxy ou le groupe $=CH-CH_2OH$.

8. Procédé selon l'une des revendications 1 à 7, caractérisé en ce que l'on convertit un composé de formule générale

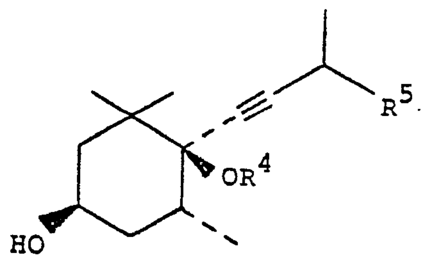

(Ia)

dans laquelle $R^5$ représente un groupe hydroxy ou le groupe $=CH-CH_2-OH$ et $R^4$ a les significations indiquées dans la revendication 1, ou ses antipodes optiques, en présence d'un composé du lithium basique faisant l'objet d'un empêchement stérique.

9. Procédé selon l'une des revendications 1 à 8, caractérisé en ce que l'on convertit un composé de formule I ou Ia dans laquelle $R^4$ représente un groupe de formule $-CO-OR^6$ ou $-CO-R^6$, de préférence $-CO-OCH_3$, $-CO-OC_2H_5$ ou $-CO-C_6H_5$.

10. Composés de formule générale

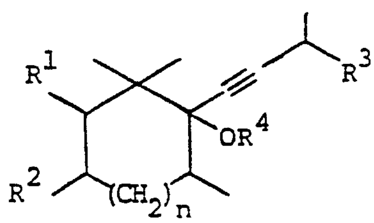

dans laquelle n est égal à 0 ou 1 ; l'un des symboles $R^1$ et $R^2$ représente l'hydrogène et l'autre l'hydrogène, un groupe hydroxy ou oxo ou un groupe hydroxy ou oxo protégé ; $R^3$ représente un groupe hydroxy, oxo, $=CH-CH_2-OH$ ou $=CH-CHO$ ou un groupe dérivé de ces derniers dont la fonction hydroxy ou oxo est protégée ; $R^4$ représente $-CO-OR^6$, $-CO-R^6$, $-CO-NR^6R^7$, $-CO-Cl$ ou $-SO_2-R^6$ ; et $R^6$ et $R^7$ représentent des radicaux hydrocarbonés saturés ou aromatiques, $R^7$ pouvant également représenter l'hydrogène.

**Revendications** (pour l'Etat contractant AT)

1. Procédé pour la préparation de cycloalcénylalcynes, caractérisé en ce que l'on convertit un composé de formule générale

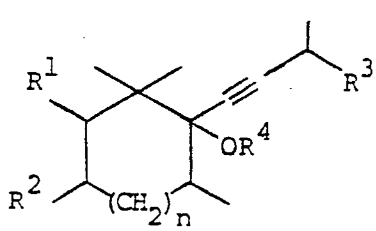

(I)

dans laquelle n est égal à 0 ou 1 ; l'un des symboles $R^1$ et $R^2$ représente l'hydrogène et l'autre l'hydrogène, un groupe hydroxy ou oxo ou un groupe hydroxy ou oxo protégé ; $R^3$ représente un groupe hydroxy, le groupe oxo, un groupe $=CH—CH_2OH$ ou $=CH—CHO$ ou un groupe dérivé de ces derniers, à fonction hydroxy ou oxo protégée ; $R^4$ représente $—CO—OR^6$, $—CO—R^6$, $—CO—NR^6R^7$, $—CO—Cl$ ou $—SO_2—R^6$ ; et $R^6$ et $R^7$ représentent des radicaux hydrocarbonés saturés ou aromatiques, $R^7$ pouvant également représenter l'hydrogène, par scission de $R^4OH$, en un composé de formule générale

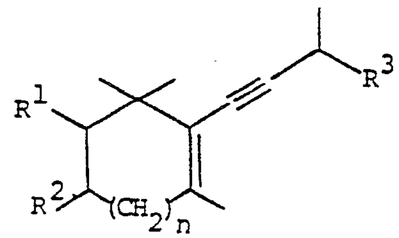

(II)

dans laquelle n, $R^1$, $R^2$ et $R^3$ ont les significations indiquées ci-dessus, et si on le désire, on hydrolyse un composé de formule II dans laquelle $R^1$, $R^2$ ou $R^3$ représente ou contient un groupe hydroxy ou oxo protégé, en le composé correspondant à groupe hydroxy ou oxo libre.

2. Procédé selon la revendication 1, caractérisé en ce que la scission de $R^4OH$ est provoquée par chauffage.

3. Procédé selon la revendication 1 ou 2, caractérisé en ce que la conversion du composé de formule I est réalisée en présence d'un catalyseur.

4. Procédé selon l'une des revendications 1 à 3, caractérisé en ce que la conversion est effectuée en présence d'une base organique azotée ou d'un sel d'une telle base ou d'un sel de lithium, de préférence en présence de l'imidazole, du 1,2,4-triazole, de l'aniline, du p-toluène-sulfonate de pyridinium, du chlorure de lithium, du perchlorate de lithium, du tert-butylate de lithium ou du tétrafluoborate de lithium.

5. Procédé selon l'une des revendications 1 à 4, caractérisé en ce que l'on convertit un composé de formule I dans laquelle n est égal à 1 et $R^1$ représente l'hydrogène.

6. Procédé selon l'une des revendications 1 à 5, caractérisé en ce que l'on convertit un composé de formule I dans laquelle l'un des symboles $R^1$ et $R^2$ représente l'hydrogène et l'autre l'hydrogène ou un groupe hydroxy, de préférence un groupe hydroxy.

7. Procédé selon l'une des revendications 1 à 6, caractérisé en ce que l'on convertit un composé de formule I dans laquelle $R^3$ représente un groupe hydroxy ou le groupe $=CH—CH_2—OH$.

8. Procédé selon l'une des revendications 1 à 7, caractérisé en ce que l'on convertit un composé de formule générale

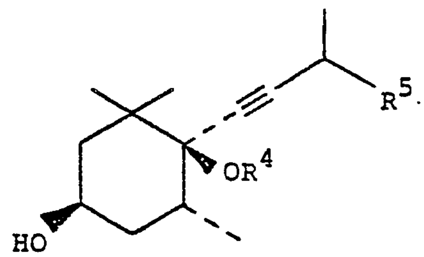

(Ia)

dans laquelle $R^5$ représente un groupe hydroxy ou le groupe $=CH—CH_2—OH$ et $R^4$ a les significations indiquées dans la revendication 1, ou ses antipodes optiques, en présence d'un composé du lithium basique faisant l'objet d'un empêchement stérique.

9. Procédé selon l'une des revendications 1 à 8, caractérisé en ce que l'on convertit un composé de formule I ou Ia dans laquelle $R^4$ représente un groupe de formule $—CO—OR^6$ ou $—CO—R^6$, de préférence $—CO—OCH_3$, $—CO—OC_2H_5$ ou $—CO—C_6H_5$.